# EUROPÄISCHE PATENTSCHRIFT

(11) **EP 1 654 993 B1**
(45) Veröffentlichungstag und Bekanntmachung des Hinweises auf die Patenterteilung: **02.01.2008**
(21) Anmeldenummer: 05014599.4
(22) Anmeldetag: 06.07.2005
(51) Int. Cl.: A61B 17/72

(54) **Intramedullärer Verriegelungsnagel**
Intramedullary locking nail
Clou de verrouillage intermedullaire

(30) Priorität: 15.09.2004 DE 202004014288 U
(43) Veröffentlichungstag der Anmeldung: 10.05.2006
(73) Patentinhaber: Stryker Trauma GmbH, 24232 Schönkirchen (DE)
(72) Erfinder: Prien, Ole, 24145 Kiel (DE)
(74) Vertreter: Maiwald Patentanwalts GmbH

(56) Entgegenhaltungen:
- EP-A- 0 736 286
- EP-A- 0 976 365
- EP-A- 1 557 131
- US-A1- 2003 195 515

## Beschreibung

Die Erfindung bezieht sich auf einen intramedullären Verriegelungsnagel mit einer Knochenschraube zur Versorgung von Knochenfrakturen nach dem Anspruch 1.

Verriegelungsnägel dienen bekanntlich zur Versorgung von Knochenfrakturen von Röhrenknochen, d.h. insbesondere des Femurs und der Tibia. In den Endbereichen des Verriegelungsnagels ist jeweils mindestens ein Querloch vorgesehen für die Durchführung einer Knochenschraube. Dadurch sind die Frakturfragmente sowohl in Torsions- als auch in Achsrichtung gesichert.

Bei Verriegelungsnägeln unterscheidet man zwischen statischer und dynamischer Verriegelung. Bei der statischen Verriegelung sind die Knochenschrauben in Kreislöchern oder -bohrungen des Nagels aufgenommen. Häufig kommt es bei dem Zusammenwachsen der Knochenfragmente aufgrund eines Sinterungsprozesses zu Verkürzungen. Da zwischen Verriegelungsnagel und Knochen eine starre Verbindung besteht, können die Knochenfragmente nicht nachgeben. Es besteht ggf. die Gefahr, daß die Knochenfragmente nicht fest zusammenwachsen.

Um der beschriebenen Erscheinung entgegenzuwirken, ist die sogenannte dynamische Verriegelung bekannt geworden. Zu diesem Zweck ist mindestens ein Querloch als Langloch ausgebildet, so daß die Knochenschraube mit dem Knochenfragment relativ zum Nagel eine bestimmte Strecke wandern kann. Eine dynamische Verriegelung ist auch dann vorzusehen, wenn dem Verriegelungsnagel Mittel zur Kompression zugeordnet sind. Mit deren Hilfe werden die Knochenfragmente gegeneinander gepreßt, nachdem der Verriegelungsnagel eingebracht worden ist.

Die EP 0 736 286 A2 offenbart einen Verriegelungsnagel, der in einem distalen Bereich Langlöcher für eine dynamische Verriegelung aufweist.

Der Erfindung liegt die Aufgabe zugrunde, einen intramedullären Verriegelungsnagel zur Versorgung von Knochenfrakturen zu schaffen, mit dem wahlweise eine statische oder eine dynamische Verriegelung erfolgen kann.

Diese Aufgabe wird durch die Merkmale des Anspruchs 1 gelöst.

Beim erfindungsgemäßen Verriegelungsnagel ist ein Endabschnitt des Langloches mit einer größeren Breite zu versehen, als ein anschließender Abschnitt des Langloches. Eine Knochenschraube hat eine Querschnittskontur und der Endabschnitt eine Kontur, die so geformt sind, daß die Knochenschraube in einer Drehstellung im Endabschnitt verriegelt ist und in einer zweiten Drehstellung ungehindert vom Endabschnitt in den anschließenden Abschnitt eintreten kann.

Bei der Erfindung hat mithin der Querschnitt der anzuwendenden Knochenschraube eine von der Kreisform abweichende Kontur und mithin unterschiedliche Durchmesser bzw. Breiten des Querschnittprofils. In einer Orientierung ist die Breite derart, daß sich die Knochenschraube in den an dem Endabschnitt anschließenden Abschnitt hinein bewegen kann. Eine andere Breite ist hingegen größer als die Breite des anschließenden Abschnitts, so daß die Knochenschraube im Endabschnitt verriegelt ist. Der Endabschnitt hat daher quer zur Längserstreckung des Langlochs eine größere Breite als der sich an den Endabschnitt anschließende Abschnitt des Langloches.

Der erfindungsgemäße Nagel ermöglicht die Wahl zwischen statischer oder dynamischer Verriegelung, was abhängig ist von der Drehstellung der Knochenschraube nach ihrem Einschrauben in den Knochen und durch das Langloch hindurch. Zu diesem Zweck kann die Knochenschraube am Kopf eine Markierung aufweisen, wodurch der Chirurg erkennen kann, ob er eine dynamische oder statische Verriegelung eingestellt hat. Es ist im übrigen auch möglich, eine zunächst statische Verriegelung in eine dynamische zu verwandeln, indem zu einem späteren Zeitpunkt die Knochenschraube um einen vorgegebenen Winkel verdreht wird, so daß nunmehr eine Relativbewegung zwischen Knochenschraube und Nagel möglich ist. So ist es schließlich denkbar, zu Kompressionszwecken zunächst eine statische Verriegelung einzustellen und nach Beendigung des Kompression auf eine dynamische Verriegelung "umzuschalten".

Es sind verschiedene Querschnittskonturen von Knochenschraube und Endabschnitt des Langloches denkbar, um die beschriebene Wirkung herbeizuführen. Eine besonders einfache Ausgestaltung der Erfindung sieht hierzu vor, daß der Endabschnitt von einer kreisförmigen Kante begrenzt ist, deren Mittelpunkt gegenüber der Längsachse des anschließenden Abschnitts quer versetzt liegt derart, daß eine Längskante des anschließenden Abschnitts eine Tangente an den Kreis bildet. Der Durchmesser des Kreises ist dabei größer als die Breite des anschließenden Abschnitts. Bei dieser Lochform braucht der Querschnitt der Knochenschraube lediglich eine Abflachung aufzuweisen, wobei die Breite des Querschnitts senkrecht zur Abflachung minimal kleiner ist als die Breite des anschließenden Abschnitts. Eine Knochenschraube mit dieser Kontur läßt sich noch relativ einfach fertigen und auch wirksam in den Knochen einschrauben.

Bei einer alternativen Ausgestaltung der Erfindung ist der Endabschnitt ebenfalls kreisförmig, wobei jedoch sein Mittelpunkt auf der Längsachse des anschließenden Abschnitts des Langlochs liegt. In diesem Fall hat der Querschnitt der Knochenschraube zwei diametral gegenüberliegende Abflachungen, deren senkrechter Abstand der Breite des anschließenden Abschnitts des Langlochs entspricht. Auch diese Form läßt sich noch relativ einfach fertigen, führt jedoch zu einer größeren Querschnittsabschwächung der Knochenschraube als bei einer einzigen Abflachung.

Die Erfindung soll nachfolgend anhand eines in Zeichnungen dargestellten Ausführungsbeispiels näher erläutert werden.
- Fig. 1: zeigt einen Abschnitt eines Verriegelungsnagels mit den erfindungsgemäßen Merkmalen.
- Fig. 2: zeigt die gleiche Ansicht wie Fig. 1, jedoch mit dynamischer Verriegelung.
- Fig. 3: zeigt eine ähnliche Darstellung wie Fig. 2, jedoch bei wirksam werdender dynamischer Verriegelung.
- Fig. 4: zeigt eine perspektivische Darstellung eines Verriegelungsnagels mit einer erfindungsgemäßen Knochenschraube.
- Fig. 5: zeigt die Darstellung nach Fig. 4 mit eingesetzter Knochenschraube, vergleichbar der schematischen Ansicht nach Fig. 2.
- Fig. 6: zeigt vergrößert in Seitenansicht den Verriegelungsnagel nach Fig. 4.
- Fig. 7: zeigt in einer anderen Ansicht den Verriegelungsnagel nach Fig. 6.
- Fig. 8: zeigt eine ähnliche Darstellung wie Fig. 5, jedoch in einer anderen Position der Knochenschraube.
- Fig. 9: zeigt die gleiche Darstellung wie Fig. 8, jedoch bei einer Verdrehung der Knochenschraube.
- Fig. 10: ist eine ähnliche Darstellung wie die Fign. 8 und 9, jedoch bei einer Relativverschiebung zwischen Verriegelungsnagel und Knochenschraube entsprechend Fig. 3.

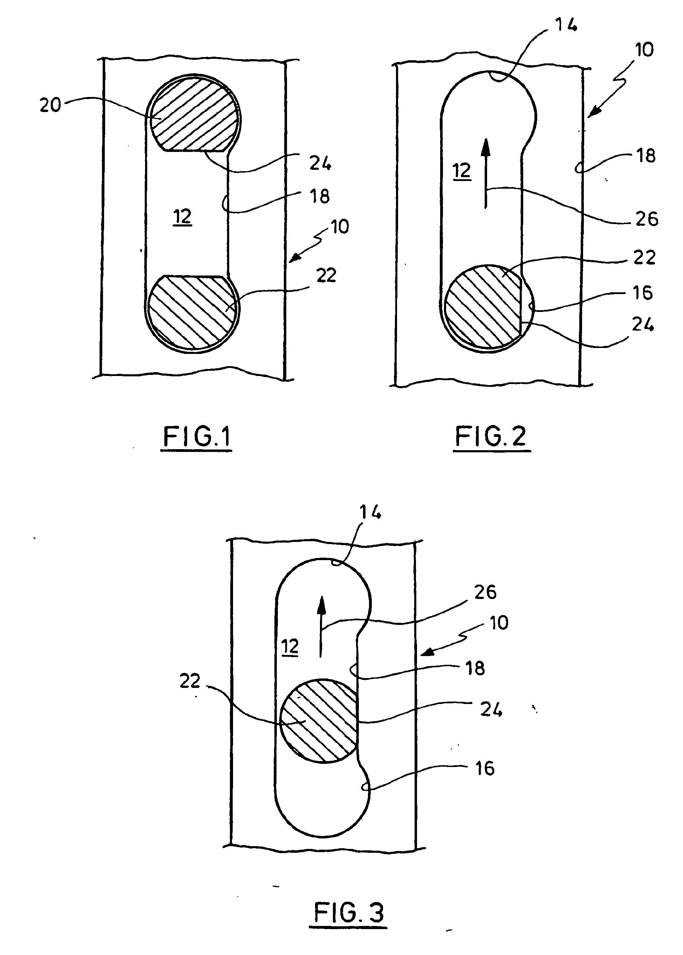

In den Fign. 1 bis 3 ist ein Verriegelungsnagel 10 angedeutet, beispielsweise für einen Femur. Das obere Ende ist nach proximal und das untere nach distal gerichtet, wobei es gleich sein soll, ob das proximale Ende oder das distale Ende des Verriegelungsnagels gezeigt ist. Bekanntlich ist sowohl am distalen als auch am proximalen Ende eines Verriegelungsnagels mindestens eine Querbohrung oder ein Querloch vorgesehen zur Aufnahme einer Knochenschraube.

Der Verriegelungsnagel 10 weist ein Langloch 12 auf, das an den Enden von einer kreisbogenförmigen Kontur begrenzt ist, wie bei 14 bzw. 16 angedeutet. Zwischen den Endabschnitten befindet sich ein Verbindungsabschnitt mit parallel beabstandeten Kanten 18. Man erkennt aus den Figuren, daß die linke Kante eine Tangente bildet an die Kreiskanten 14, 16. Der Mittelpunkt der Kreiskanten 14, 16 liegt versetzt zur Längsachse des Verbindungsabschnitts, und der Durchmesser der Kreiskanten 14, 16 ist größer als der Abstand der Kanten 18 des Verbindungsabschnitts. In Fig. 1 ist das Querschnittprofil von zwei Knochenschrauben 20, 22 dargestellt, die jeweils in den Endabschnitten des Langloches 12 angeordnet sind. Der Durchmesser der Knochenschrauben 20, 22 ist minimal kleiner als der Durchmesser der Kreiskanten 14, 16. Wie ferner zu erkennen, weisen die Knochenschraube 20, 22 im Querschnitt eine Abflachung 24 auf. In den in Fig. 1 dargestellten Drehstellungen der Knochenschrauben 20, 22 sind diese in den Endabschnitten "verriegelt". Mit anderen Worten, sie können bei einer axialen Relativkraft zwischen Nagel 10 einerseits und Knochenschraube 20, 22 andererseits sich nicht bewegen.

In Fig. 2 ist die Knochenschraube 20 weggelassen und die Knochenschraube 22 um 90° verdreht. Man erkennt, daß der Durchmesser der Knochenschraube 22 senkrecht zur Abflachung 24 minimal kleiner ist als der Abstand der Längskanten 18. Nunmehr kann eine Relativbewegung zwischen Langloch 12 und Knochenschraube 22 stattfinden, wie dies in Fig. 3 dargestellt ist. Die Bewegungsrichtung ist durch Pfeil 26 angedeutet.

Es versteht sich, daß die in den Figuren dargestellten Querschnitte der Knochenschrauben 20, 22 den Schaftquerschnitt wiedergibt und der Kopf der Knochenschraube 20, 22 im Durchmesser größer sein kann zwecks wirksamer Anlage an die Kortikalis.

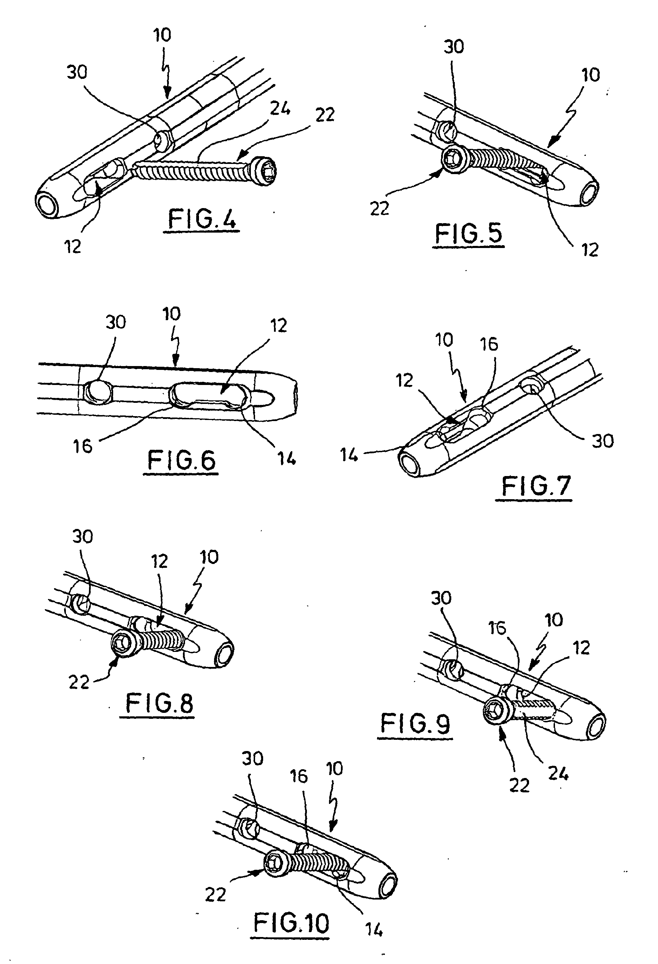

In den Fign. 4 bis 10 sind Verriegelungsnagel 10 und Knochenschraube 22 gemäß den Fign. 1 bis 3 perspektivisch dargestellt. Man erkennt das distale bzw. proximale Ende des Verriegelungsnagels, je nachdem, wie er zur Versorgung in den Knochen eingesetzt wird. Das jeweils andere Ende des Verriegelungsnagels 10 ist nicht dargestellt. Es kann herkömmlich gestaltet sein.

Man erkennt ferner, daß neben dem speziell gestalteten Langloch 12, das, wie beschrieben, sich durch den Nagel 10 quer hindurcherstreckt, eine kreisrunde Öffnung 30 vorgesehen ist, durch welche ebenfalls eine Knochenschraube hindurchgeführt werden kann. Diese muß jedoch nicht wie die Schrauben 20, 22 mit einer Abflachung 24 versehen werden. In Fig. 5 befindet sich die Knochenschraube 22 im erweiterten Bereich 16 des Langloches 12 und in Fign. 8 und 9 im erweiterten Bereich 14 des Langloches 12. In den Fign. 8 und 10 ist die Abflachung 24 (nicht zu sehen) nach unten vorgesehen, so daß, wie in Fig. 10 gezeigt, eine Bewegung relativ von Nagel 10 und Knochenschraube 22 möglich ist. In Fig. 9 hingegen ist eine Verriegelung zwischen Knochennagel 10 und Knochenschraube 12 gezeigt, so daß eine Relativbewegung zwischen diesen Teilen verhindert ist.

## Patentansprüche

1. Intramedullärer Verriegelungsnagel mit einer knochenschraube zur Versorgung von Knochenfrakturen, wobei der Verriegelungsnagel (10), in beabstandeten Bereichen jeweils mindestens ein Querloch aufweist für die Aufnahme der Knochenschraube, wobei mindestens ein Querloch als ein sich in Achsrichtung erstreckendes Langloch ausgebildet ist, **dadurch gekennzeichnet, daß** ein Endabschnitt des Langloches (12) eine größere Breite als ein anschließender Abschnitt des Langloches (12) hat, wobei der anschließende Abschnitt über seine Länge eine konstante Breite aufweist und die Knochenschraube (20, 22) in ihrer Querschnittskontur und der Endabschnitt so geformt sind, daß die Knochenschraube in einer ersten Drehstellung im Endabschnitt verriegelt ist und in einer zweiten Drehstellung ungehindert vom Endabschnitt in den anschließenden Abschnitt eintreten kann.

2. Verriegelungsnagel nach Anspruch 1, **dadurch gekennzeichnet, daß** beide Endabschnitte des Langlochs (12) identisch ausgebildet sind.

3. Verriegelungsnagel nach Anspruch 1 oder 2, **dadurch gekennzeichnet, daß** der Endabschnitt von einer kreisförmigen Kante (14, 16) begrenzt ist, deren Mittelpunkt gegenüber der Längsachse des anschließenden Abschnitts quer versetzt liegt derart, daß eine Längskante des anschließenden Abschnitts eine Tangente an den Kreis bildet, wobei der Durchmesser des Kreises größer ist als die Breite des anschließenden Abschnitts und der Querschnitt der Knochenschraube (20, 22) eine Abflachung (24) aufweist, wobei die Abmessung des Querschnitts senkrecht zur Abflachung (24) der Breite des anschließenden Abschnitts des Langlochs (12) entspricht.

4. Verriegelungsnagel nach Anspruch 1 oder 2, **dadurch gekennzeichnet, daß**-der Endabschnitt von einer kreisförmigen Kante begrenzt ist, deren Mittelpunkt auf der Längsachse des anschließenden Abschnitts des Langloches liegt, wobei der Durchmesser des Kreises größer ist als die Breite des anschließenden Abschnitts und der Querschnitt der Knochenschraube zwei diametral gegenüberliegende Abflachungen aufweist, deren senkrechter Abstand der Breite des anschließenden Abschnitts des Langlochs entspricht.

## Claims

1. Intramedullary locking nail with a bone screw for treating bone fractures wherein the locking nail (10) has spaced-apart regions each comprising at least one cross hole for receiving the bone screw, wherein at least one cross hole is embodied as an elongated hole extending in the axial direction, **characterised in that** an end portion of the elongated hole (12) is wider than an adjacent portion of the elongated hole (12), wherein the adjacent portion has a constant width over its length and the cross-sectional contour of the bone screw (20, 22) and the end portion are formed such that the bone screw is locked in a first rotational position in the end portion and in a second rotational position can enter the adjacent portion unimpeded from the end portion.

2. Locking nail according to claim 1, **characterised in that** the two end portions of the elongated hole (12) have an identical design.

3. Locking nail according to claim 1 or claim 2, **characterised in that** the end portion is bounded by a circular edge (14, 16), the mid point of which is located transversely offset relative to the longitudinal axis of the adjacent portion in such a way that a longitudinal edge of the adjacent portion forms a tangent to the circle, wherein the diameter of the circle is greater than the width of the adjacent portion and the cross section of the bone screw (20, 22) comprises a flat segment (24), wherein the dimension of the cross section perpendicular to the flat segment (24) corresponds to the width of the adjacent portion of the elongated hole (12).

4. Locking nail according to claim 1 or claim 2, **characterised in that** the end portion is bounded by a circular edge, the mid point of which lies on the longitudinal axis of the adjacent portion, wherein the diameter of the circle is greater than the width of the adjacent portion and the cross section of the bone screw comprises two diametrically opposite flat segments with a perpendicular spacing corresponding to the width of the adjacent portion of the elongated hole.

## Revendications

1. Clou de verrouillage intramédullaire, comportant une vis osseuse, pour réduire des fractures osseuses, le clou de verrouillage (10) comportant dans des zones distantes chaque fois au moins un trou transversal pour recevoir la vis osseuse, au moins un trou transversal étant réalisé sous forme de trou oblong s'étendant en direction axiale, **caractérisé en ce qu'**une section d'extrémité du trou oblong (12) présente une largeur plus grande qu'une section adjacente du trou oblong (12), la section adjacente présentant une largeur constante sur sa longueur et les vis osseuses (20, 22) dans leur contour de section transversale et la section d'extrémité étant formées de telle façon que la vis osseuse dans une première position de rotation est verrouillée dans la section d'extrémité et dans une seconde position de rotation peut pénétrer sans obstacle de la section d'extrémité dans la section adjacente.

2. Clou de verrouillage selon la revendication 1, **caractérisé en ce que** les deux sections d'extrémité du trou oblong (12) sont réalisées de manière identique.

3. Clou de verrouillage selon la revendication 1 ou 2, **caractérisé en ce que** la section d'extrémité est limitée par une arête circulaire (14, 16) dont le centre est décalé transversalement par rapport à l'axe longitudinal de la section adjacente, de telle façon qu'une arête longitudinale de la section adjacente forme une tangente sur le cercle, le diamètre du cercle étant plus grand que la largeur de la section adjacente et la section transversale de la vis osseuse (20, 22) comportant un méplat (24), la dimension de la section transversale perpendiculairement au méplat (24) correspondant à la largeur de la section adjacente du trou oblong (12).

4. Clou de verrouillage selon la revendication 1 ou 2, **caractérisé en ce que** la section d'extrémité est limitée par une arête de forme circulaire, dont le centre se situe sur l'axe longitudinal de la section adjacente du trou oblong, le diamètre du cercle étant plus grand que la largeur de la section adjacente et la section transversale de la vis osseuse comportant deux méplats diamétralement opposés l'un à l'autre, dont la distance orthogonale correspond à la largeur de la section adjacente du trou oblong.
